# EUROPEAN PATENT SPECIFICATION

(11) **EP 3 069 127 B1**
(45) Date of publication and mention of the grant of the patent: **26.07.2023**
(21) Application number: 14862472.9
(22) Date of filing: 11.11.2014
(51) Int. Cl.: G01N 24/08, G01N 33/18, G01N 33/24, A01C 23/00

(54) **A METHOD FOR QUANTITATIVE DETERMINATION OF NITROGEN IN AN AQUEOUS FLUID**
VERFAHREN ZUR QUANTITATIVEN BESTIMMUNG VON STICKSTOFF IN WÄSSRIGEN FLÜSSIGKEITEN
PROCÉDÉ DE DÉTERMINATION QUANTITATIVE DE L'AZOTE DANS UN LIQUIDE AQUEUX

(30) Priority: 13.11.2013 DK 201370682
(43) Date of publication of application: 21.09.2016
(73) Proprietor: NanoNord A/S, 9220 Aalborg Ø (DK)
(72) Inventor: JENSEN, Ole Nørgaard, DK-9000 Alborg (DK); NIELSEN, Niels Christian, DK-8220 Brabrand (DK); SØRENSEN, Morten Kjærulff, DK-8200 Aarhus N (DK)
(74) Representative: Plougmann Vingtoft a/s
(86) International application number: PCT/DK2014/050380
(87) International publication number: WO 2015/070872

(56) References cited:
- WO-A1-2012/016624
- DE-A1- 19 735 927
- DE-U1-202007 000 724
- US-A- 5 314 827
- US-A- 5 817 474
- US-A1- 2003 088 364
- US-A1- 2007 055 456
- LARRY S SIMERAL: "Determination of Urea, Nitrate, and Ammonium in Aqueous Solution Using Nitrogen-14 Nuclear Magnatic Resonance", APPLIED SPECTROSCOPY, vol. 51, no. 10, 1 January 1997 (1997-01-01), pages 1585-1587, XP055368741,
- Barbara Cade-Menun ET AL: "Solution Phosphorus-31 Nuclear Magnetic Resonance Spectroscopy of Soils from 2005 to 2013: A Review of Sample Preparation and Experimental Parameters", Soil Science Society of America Journal, 1 November 2013 (2013-11-01), pages 19-37, XP055369567, Madison DOI: 10.2136/sssaj2013.05.0187dgs Retrieved from the Internet: URL:https://dl.sciencesocieties.org/public ations/sssaj/pdfs/78/1/19 [retrieved on 2017-05-04]
- D Solomon ET AL: "DIVISION S-2-SOIL CHEMISTRY Soil Organic Matter Composition in the Subhumid Ethiopian Highlands as Influenced by Deforestation and Agricultural Management", Published in Soil Sci. Soc. Am. J, 1 January 2002 (2002-01-01), pages 68-82, XP055369677, Retrieved from the Internet: URL:http://www.css.cornell.edu/faculty/leh mann/publ/SoilSciSocAmJ%2066,%2068-82,%202 002%20Solomon.pdf [retrieved on 2017-05-04]
- MITCHELL J ET AL: "Low-field permanent magnets for industrial process and quality control", PROGRESS IN NUCLEAR MAGNETIC RESONANCE SPECTROSCOPY, vol. 76, 30 September 2013 (2013-09-30), pages 1-60, XP028806298, ISSN: 0079-6565, DOI: 10.1016/J.PNMRS.2013.09.001

## Description

### TECHNICAL FIELD

The invention relates to a method for quantitative determination of nitrogen present in the form of one or more nitrogen containing compounds or ions thereof in an aqueous fluid.

### BACKGROUND ART

The presence of nitrogen containing compounds is often determined by determining other parts of the compound in question, or by determining the reaction of the compound.

The standard method for quantitative determination of nitrogen in chemical substancesis the Kjeldahl method developed by Johan Kjeldahl in 1883.

The method is a cumbersome and time consuming method comprising heating the substance with sulfuric acid to reduce and liberate the nitrogen as ammonium sulfate followed by distillation with sodium hydroxide to obtain ammonia. The amount of ammonia present, and thus the amount of nitrogen present in the sample, is determined by back titration.

Several improvements have been provided to simplify and speed up the determination including using different catalysts, replacing the back titration with other types of analysis such as potentiometric titration or electrophoresis. For reducing the man power required equipment has been developed to ensure a more or less automated Kjeldahl determination of nitrogen.

The total amount of nitrogen is often given as the TKN amount which means the sum of organic nitrogen, ammonia (NH3), and ammonium (NH4+) in the chemical analysis of soil, water, or wastewater (e.g. sewage treatment plant effluent). Today, TKN is a required parameter for regulatory reporting at many treatment plants, and as a means of monitoring plant operations.

The article "Determination of Urea, Nitrate, and Ammonium in Aqueous Solution Using Nitrogen-14 Nuclear Magnetic Resonance" by Larry S. Simeral, Applied Spectroscopy, Vol. 51, number 10, 1997, pages 1585-1587 describes a test setup for quantitative determination of urea, nitrate and ammonium diluted in water using an NMR instrument and N-14NMR is described wherein deuterium and acetonitrile are added to the sample. Even though the known methods have been improved and they are still rather time consuming and costly. Further these prior art tests are very inflexible and generally require that samples are brought to the laboratory for test.

The object of the invention is to provide a new method of quantitative determination of nitrogen present in the form of one or more nitrogen containing compounds or ions thereof in an aqueous fluid which method is both fast and reliable and may be used in a very flexible way e.g. by performing the measurements on site and/or in-line .

### DISCLOSURE OF INVENTION

This object has been solved by the present invention as defined in the claims. The method or the system of the invention for quantitative determination of nitrogen present in the form of one or more nitrogen containing compounds or ions thereof in an aqueous fluid and/or embodiments thereof has shown to have a large number of advantages, which will be clear from the following description.

As explained above, before the present invention was conceived quantitative determination of nitrogen present in the form of one or more nitrogen containing compounds or ions thereof in an aqueous fluid was very difficult and cumbersome and/or generally required a destructive test.

The present invention provides a highly improved, very reliable, fast and nondestructive method which is, can measure on large amounts of the aqueous fluid which in practice means that the method of the invention is also applicable on highly in-homogeneous aqueous fluids. Further, the method of the invention can be used in a very flexible way e.g. by performing the measurements on site and/or in-line or by taking suitable samples.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Reference made to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the skilled person will understand that particular features, structures, or characteristics may be combined in any suitable manner within the scope of the invention as defined by the claims.

The term "substantially" should herein be taken to mean that ordinary product variances and tolerances are comprised.

Surprisingly it has been found that quantitative determination of nitrogen present in the form of one or more nitrogen containing compounds or ions thereof in an aqueous fluid as defined in the claims can be conducted in a simple and fast way while simultaneously having a very high reliability in particular where the nitrogen containing compounds or ions thereof are relatively small, such as 500 Dalton or less.

The method of the invention comprises quantitative determination of nitrogen present in the form of one or more nitrogen containing compounds or ions thereof of in an aqueous fluid. The method comprises subjecting at least a part of the aqueous fluid to an NMR reading comprising generating a ¹⁴N data comprising a ¹⁴N NMR data spectra and correlating the ¹⁴N NMR data to calibration data. According to the method of the invention the NMR reading is performed in a magnetic field of up to about 2.5 Tesla.

The generally known NMR spectroscope operates with magnetic fields B of from about 10 Tesla and higher. Heretofore it has generally been believed that the higher the magnetic field the higher resolution can be obtained, and normally NMR spectroscope has been applied as large and expensive laboratory equipment. The inventor of the present invention has surprisingly found that by applying a low field ¹⁴N NMR determination using a magnetic field B of up to about 2.5 Tesla a very accurate determination can be obtained at low cost - low equipment cost - and simultaneously the determination can be performed very fast - i.e. within minutes - or even seconds.

Advantageously, the NMR reading is performed in a magnetic field of from about 0.3 Tesla to about 1.5 Tesla. Due to this relatively low magnetic field the equipment for performing the NMR reading can be kept at a surprisingly low cost while simultaneously a high resolution can be obtained.

In the article "Determination of Urea, Nitrate, and Ammonium in Aqueous Solution Using Nitrogen-14 Nuclear Magnetic Resonance" by Larry S. Simeral, Applied Spectroscopy, Vol. 51, number 10, 1997, pages 1585-1587 a test setup for quantitative determination of urea, nitrate and ammonium diluted in water using an NMR instrument and N-14NMR is described. However, the described test setup has never been applied in practice, possibly because the described test was relatively expensive to perform and difficult to obtain results with high accuracy.

It has even been found that when performing the NMR reading in a low magnetic field according to the invention, addition of reference components is not required and in fact not decired. It has been found that very fast and accurate determinations can be obtained without any reference components in the aqueous sample. This is very beneficial both because such reference components add to the cost of performing the determination and also because addition of such reference components results in a contamination of the aqueous fluid which may prevent using the aqueous fluid e.g. for fertilizer after the determination has been performed. In the aqueous fluid in the method of the invention no deuterium oxide and acetonitrile is added and the aqueous liquid is substantially free of deuterium oxide and acetonitrile.

In an embodiment, the aqueous fluid is substantially free of relaxation agent. In an embodiment the method does not comprise addition of relaxation agent to the aqueous fluid.

The method of the invention has further shown to be suitable even where the concentration of nitrogen is relatively high as well as rather low. It has been found that quantitative determination of one or more nitrogen compounds in an aqueous fluid can be performed with a surprising high accuracy based on NMR reading of nitrogen-14 isotopes. Heretofore NMR has generally been used as a means for determine the presence or absence of selected compounds i.e. qualitative determinations.

Natural nitrogen (N) consists of two stable isotopes, nitrogen-14 (herein referred to as ¹⁴N) and nitrogen-15 (herein referred to as ¹⁵N). ¹⁵N NMR detection has frequently been applied in agricultural and medical research for qualitative determinations in particular for DNA analysis. Nitrogen-15 has further been used to trace mineral nitrogen compounds (particularly fertilizers) in the environment and when combined with the use of other isotopic labels, is also a very important tracer for describing the fate of nitrogenous organic pollutants. In practice ¹⁴N NMR analysis has only rarely been used even though the nitrogen-14 isotope is much more frequent than the nitrogen-15 isotope. The main reason for this is that the nitrogen-14 is quadrupolar with 7 protons and 7 neutrons, each contributing with a nuclear spin of plus or minus spin 1/2, thereby resulting in a total magnetic spin of one. Generally quadrupolar nuclei are much more difficult to determine than symmetric nuclei with spin-½ such as ¹H which is the most frequent nuclei used in NMR determinations. When exiting energy is added to quadrupolar nuclei in a magnetic field, their energies will split into multiple levels. Further splitting patterns can be seen in their coupling with other nuclei. The NMR signals of quadrupolar nucleic are usually wider than those of spin-½ nuclei due to rapid quadrupolar relaxation. The line-width increases with the line-width factor that is related to the quadrupole moment of the nucleus, the size and asymmetry of the component. "Chemical Shifts of 14N in the NMR Spectra of Ammonia and Related Compounds" by Bernhard M. Schmidt, Journal of Molecular Spectroscopy 2, 539-550 (1958) describes a study of the chemical shifts of ¹⁴N. In WO2013/077922 an attempt to apply ¹⁴N NMR in combination with 1H NMR in detection of specific explosives, such as nitric acid and peroxide in closed containers is described.

Spectrometers are well known in the art and the skilled person will be able to provide a suitable spectrometer for use in the present invention based on the teaching provided herein. Examples of spectrometer are e.g. described in US 6,310,480 and in US 5,023,551.

A spectrometer comprises a unit for providing a permanent field e.g. a permanent magnet assembly as well as a transmitter and a receiver for transmitting and/or receiving RF frequency pulses/signals. The RF receiver and RF transmitter are connected to an antenna or an array of RF antennae which may be in the form of transceivers capable of both transmitting and receiving. The spectrometer further comprises at least one computing element, in the following referred to as a computer.

General background of NMR formation evaluation can be found, for example in U.S. Pat. 5,023,551.

Although 'NMR measurement' in the following often will be used in singular to describe the invention, it should be observed that the singular term 'NMR measurement' also includes a plurality of NMR measurements unless otherwise specified.

Although 'NMR reading' (or 'NMR measurement') in the following often will be used in singular to describe the invention, it should be observed that the singular term 'NMR reading' also includes a plurality of NMR readings unless other is specified.

NMR reading means performing NMR spectroscopy on the sample in question.

The terms 'NMR reading' and 'NMR Measurement' are used interchangeable.

The phrase "NMR reading time" means the total time for performing one or more NMR readings to obtain NMR data for quantitative determination of one or more nitrogen containing compounds in the aqueous fluid.

The phrase 'nitrogen containing compounds' should herein be interpreted to mean 'nitrogen containing compounds and/or ions thereof' unless otherwise specified.

The terms 'NMR reading' and 'NMR Measurement' are used interchangeably.

Preferably, the method comprises quantitative determination of one or more nitrogen containing compounds by determining the content of the one or more nitrogen containing compounds or ions thereof in the aqueous fluid. The content of one or more nitrogen containing compounds can be provided in the form of number of nitrogen atoms or compounds as well as concentration thereof in number or weight or in any other quantitative unit. In an embodiment, the method comprises determining the total amount of nitrogen atoms. In an embodiment, the method comprises determining the total amount of nitrogen containing compounds. In an embodiment, the method comprises determining the total amount of at least two different nitrogen containing compounds.

'A nitrogen containing compound' is herein used to denote a chemical substance comprising one or more nitrogen atoms and at least one other different chemical element, which are chemically bonded to each other or ions thereof. Nitrogen compounds comprise molecular compounds held together by covalent bonds, salts held together by ionic bonds or ions thereof, or complexes held together by coordinate covalent bonds. Pure chemical nitrogen is not considered to be a compound.

It has been found that the method of the invention especially is highly reliable for use in the quantitative determination when the one or more nitrogen containing compounds are dissolved or dispersed in the aqueous fluid. Advantageously the aqueous fluid is substantially free of nitrogen containing precipitates and/or sediments. Advantageously the aqueous fluid is free of precipitates and/or sediments. Where the aqueous fluid comprises small amounts of precipitates and/or sediments it is advantageously to subject the aqueous fluid to turbulence during the NMR reading for improving the quantitative accuracy.

In an embodiment, the nitrogen concentration in the aqueous fluid is at least about 4 wt-%, such as at least about 5-wt-%, such as at least about 5.5 wt-%.

The method of the invention has shown to be very useful for quantitative determination(s) of nitrogen containing compounds in the form of inorganic compounds or in the form of a combination of inorganic and organic compounds.

The method of the invention is extremely reliable where the nitrogen containing compounds are relatively small and in fact it has been found that for nitrogen containing compounds having a molecular weight of up to about 200 Da, the quantitative determination may be superior both with respect to speed and accuracy to prior art quantitative determinations of similar compounds. In an embodiment, the method therefore comprises quantitative determination of nitrogen in aqueous fluid where the one or more nitrogen containing compounds have a molecular weight of up to about 200 Da, such as from about 15 to about 100 Da. Generally it is most preferred that the one or more nitrogen containing compounds have a molecular weight of from about 15 Da to about 200 Da.

In an embodiment the method comprises quantitative determination of Nitrite (NO₂⁻), Nitrate (NO₃⁻), Melamine (C₃H6N6), Urea ((NH₂)₂CO), NH₃, NH₄⁺, or any combinations thereof.

A particular advantage of the method of the invention has found to be that it can be applied to determine the relative amount of Nitrate (NO₃⁻) versus ammonia (NH₃ and NH₄⁺) and/or to determine the relative amounts of NH₃ versus NH4⁺. For any other analytic tools such relative determination is rather time consuming and often requires steps of reacting a sample with different chemicals which not only are a source for errors but also result in a destructive analysis.

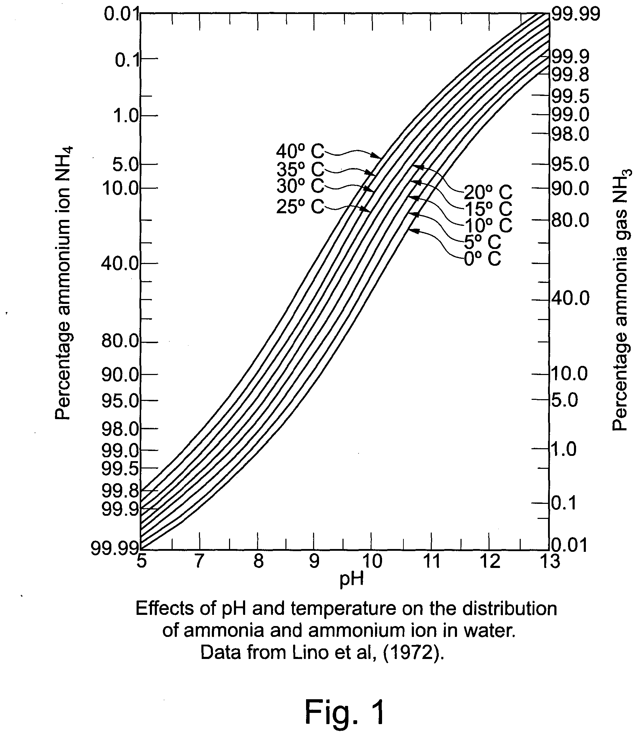

In many situations, the relative amounts of NH₃ versus NH4⁺ are a very important knowledge. It has been found the relative amounts of NH₃ versus NH4⁺ largely depend on the pH value of the aqueous fluid. Thereby by determining the relative amounts of NH₃ versus NH4⁺ the pH value may be determined. Also, the temperature has a certain influence on the pH value. This will be described further below with reference to Fig. 1.

By performing a number of NH₃ versus NH4⁺ determinations at different temperatures using the method of the invention the pH value can be determined with a very high accuracy.

In an embodiment where the method comprises measuring the temperature, the measured temperature of at least part of the aqueous fluid and the relative measured contents of NH₃ versus NH4⁺ are used to calculate and display the pH value in the range 5.5 to 12. The temperature may e.g. be measured using a digital temperature sensor.

The nitrogen containing compounds of the aqueous fluid preferably have molecular weight of 200 Da or less.

In an embodiment, the aqueous fluid is or comprises a mammal body fluid, such as saliva, blood or urine. The method of the invention has been found to be extremely useful in analyzing urine. In an embodiment the method of the invention is used as a diagnostic tool wherein the method is applied to determine the amounts of nitrogen components in a patient's urine for providing data to the doctor for determining the status of the patient e.g. for determining if the patient is in good health or has a certain disease. It has been found that the method of the invention may also be used as a part of a doping or drug test since many doping compositions or drugs have influence on the balance of the nitrogen containing compositions in urine of a user.

In a preferred embodiment the method of the invention comprises quantitative determination of nitrogen present in the form of one or more nitrogen containing compounds or ions thereof in a liquid manure and subjecting at least a part of the manure to an NMR reading comprising generating a ¹⁴N data comprising a ¹⁴N NMR data spectra and correlating the ¹⁴N NMR data to calibration data.

The method of the invention where the aqueous fluid is liquid manure has extremely high economic potential. Generally, farmers today have many difficulties in disposing of the liquid manure produced by their livestock animals such as from horses, goats, sheep, pigs, cows, ducks and poultry. To avoid environment pollution and over fertilization uncontrolled manure emission is generally banned in most countries and manure spreading on farming soil is generally highly regulated. Both small scale livestock producers as well as large scale livestock producers are faced with many costly challenges when managing their manure according to regulation. Disposal of manure according to regulations requires a constant control of the nutrition, such as the NPK values in the manure, a detailed control of the amount of manure, which can be spread onto the fields or disposed of by other means. Heretofore the standard method has been to withdraw samples of the manure and sending the samples to chemical analysis in a remote laboratory. By using the method of the invention the amount of nitrogen containing compounds can easily be retrieved and simultaneously the composition e.g. relative amounts of one or more nitrogen components can be determined. It has been found that the NMR equipment simultaneously can be used to perform quantitative determination of other nutrients in the manure such a phosphor containing components and potassium containing composition and accordingly the method of the invention may advantageously additional comprise quantitative determinations of phosphor and/or potassium. Thereby the determination of the nutrients in the manure can be performed very fast and preferably on site i.e. there is no need to send samples to a remote laboratory. An additional benefit of the method of the invention is that the aqueous fluid e.g. the manure needs not be in contact with expensive equipment. The NMR reading of the aqueous fluid can for example be performed on the aqueous fluid in a non-corrosive container e.g. polymer container or a polymer pipe.

In an embodiment, the method therefore further comprises quantitative determination of at least one phosphor containing component, preferably by subjecting at least a part of the aqueous fluid to an NMR reading comprising generating a ³¹P data and correlating the ³¹P NMR data to calibration data.

In an embodiment the method therefore further comprises quantitative determination of at least one inorganic potassium containing ion, preferably by subjecting at least a part of the aqueous fluid to an NMR reading comprising generating a ³⁹K data and correlating the ³⁹K NMR data to calibration data.

Since, as described above, the pH value of the aqueous fluid can be determined from the relative amounts of NH₃ versus NH4⁺, the method of the invention where the aqueous fluid is liquid manure preferably also comprises determination of the pH value. In general, a requirement for storing manure is that the manure must not be too acidic e.g. it must not have a pH value of 5.5 or less due to gaseous emissions. Further very acidic manure may result in corrosion of equipment, containers and other.

By obtaining an accurate determination of the amount of nitrogen components in the manure and optionally other nutrients the optimal amount of manure can be spread onto the field for soil fertilization to thereby optimize the fertilization of the soil.

Today the limiting nutrient is often the amount of phosphor containing components in the manure. However, a large amount of the nitrogen containing components can be removed using a simple filtration and the filtrate can be used for production of dry matter which is both simple to store and can be used for fertilizer or for incineration. In the liquid slurry after filtration, the limiting nutrient is usually the nitrogen containing component.

In order to avoid over fertilization of the soil which may likely lead to bleeding into lakes and water streams - which may damage the life in such lakes and water streams - the amount of manure which is spread is usually less than what would have been the optimal had the farmer known the exact amount of nitrogen containing components and optionally the amount of other nutrients. By using the method of the present invention, the farmer can determine the amounts of nitrogen containing components and optionally the amount of other nutrients with a very high accuracy and thereby the fertilization of the soil can be optimized. The amount of fertilizer allowed on a field depends largely on the amount of nutrients in the soil of the field as well as on what kind of crop is about to be grown on the field. The allowed amount of nutrients, which can be spread by the manure is therefore different from field to field.

In an embodiment, the method of the invention comprises quantitative determination of nitrogen present in the form of one or more nitrogen containing compounds or ions thereof in an aqueous fluid in the form of lake or sea water. Thereby it will be possible to determine very quickly if adjacent fields have been over fertilized - i.e. the farmer's spreading of manure can be determined in a simple and effective way. In an embodiment the lake or sea water is subjected to a surveillance e.g. by placing NMR detectors at selected positions where bleeding of surplus fertilizer from the fields are to be expected.

In an embodiment, the aqueous fluid is process wastewater, non-treated wastewater, treated wastewater, ground water or tap water.

The NMR reading is advantageously performed on the aqueous fluid in a flowing condition or in a semi-flowing condition.

The phrase that the NMR reading is performed on the aqueous fluid in a flowing condition means that the aqueous fluid is flowing through the magnetic field during the reading.

The phrase that the NMR reading is performed on the aqueous fluid in a semi-flowing condition means that the aqueous fluid is flowing through the magnetic field and temporarily stopped during at least a part of the reading.

In an embodiment of the invention, the NMR measurement is performed on the aqueous fluid in a flowing condition. The NMR measurement may for example be performed on the aqueous fluid during transportation from a first fluid reservoir to a second fluid reservoir or to a point of use, such as to a storage container or to be spread onto a field e.g. from a fluid reservoir in the form of a tank arranged on a manure spreading truck to a second fluid reservoir in the form of a field to be fertilized.

In an embodiment, the NMR measurement is performed on the aqueous fluid in a flowing condition in a pipe section pumping the aqueous fluid from a first fluid reservoir e.g. a container and back to the same first fluid reservoir. In this embodiment the method can advantageously comprise continuous determination of the pH value during fermentation and/other treatment of the aqueous fluid. Thereby the condition and the development of the aqueous fluid can be monitored by monitoring the relative amount of two or more nitrogen containing components.

In an embodiment the NMR measurement is performed on the aqueous fluid in a flowing condition in a pipe section pumping the aqueous fluid from a first fluid reservoir to a second fluid reservoir e.g. to be spread to a selected point of use.

When performing the NMR measurement on the aqueous fluid in a flowing condition it should advantageously be ensured that the velocity of the flowing aqueous fluid is adjusted or kept such that the aqueous fluid part is within the spectrometer range for a sufficient time to perform the NMR measurement.

In an embodiment, the NMR measurement is performed on the aqueous fluid in a semi-flowing condition in a pipe section pumping the aqueous fluid from a first fluid reservoir to a second fluid, wherein the aqueous fluid is temporarily stopped during at least a part of the NMR reading, advantageously a preselected amount is pumped through the pipe section, followed by a temporary flow stop where after the flowing of the of is resumed.

In an embodiment of the invention, the NMR measurement is performed in-line or semi-in-line, comprising performing the NMR measurement on-site, such as on-site of a manure spreading truck.

In an embodiment the NMR measurement is performed in-line directly on the aqueous fluid in a flowing condition e.g. in a pipe preferably performing measurement on the major amount of the aqueous fluid or even all of the aqueous fluid.

The term "in-line" should herein be interpreted to mean that the NMR measurement is performed directly on the aqueous fluid source - e.g. the manure of the manure reservoir - without taking a sample or samples of the aqueous fluid and measuring on such sample(s) only. The NMR measurement may e.g. be performed on the aqueous fluid in a flowing condition as described above or it may be performed directly on the aqueous fluid in a container e.g. e.g. as described below.

In an embodiment, the method further comprises determination of the flow of the aqueous fluid in the magnet field.

The method advantageously comprises determination of the total amount of nitrogen containing compounds or ions thereof, however, for certain applications the quantitative determination of one or more nitrogen component is sufficient.

In an embodiment, the NMR measurement comprises simultaneously subjecting the aqueous fluid to a magnetic field B and a plurality of pulses of radio frequency energy E (in the form of RF pulses) and receiving electromagnetic signals from the ¹⁴N isotopes. RF pulses mean herein pulses of radio frequency energy.

According to the invention the magnetic field B is relatively low and simultaneously a high resolution (i.e. as low noise as possible) can surprisingly be obtained.

In an embodiment, the magnetic field is generated by a permanent magnet, such as a neodymium magnet. Since permanent magnets are generally not costly, this solution provides a low cost solution, which for many applications may provide a sufficiently low noise result.

In an embodiment, the magnetic field is generated by an electromagnet, such as a solenoid magnet or other electromagnets, which are usually applied in motors, generators, transformers, loudspeakers or similar equipment. Electromagnets of suitable strength e.g. electromagnets that can be applied for generating a field of about 1.5 Tesla or more are often relatively expensive compared with permanent magnets. Furthermore, the electromagnet may be adjusted by adjusting the current in the coil of the electromagnet to a desired level.

In an embodiment is generated by a permanent magnet in combination with an electromagnet, which advantageously is constructed for providing a pulsed magnetic field.

In an embodiment, the NMR reading is performed in a pulsed magnetic field. By pulsing the magnetic field, even more accurate determinations can be obtained because measurements at different field strengths provide a tool for identifying noise which may accordingly be filtered of.

Advantageously the NMR reading is performed in a magnetic field with a standard deviation of the field over the sample volume of more than 30 ppm such as from about 300 ppm to 3000 ppm. Such magnetic field will in particular be suitable for ¹⁴N NMR readings.

In an embodiment of the invention the magnetic field in the measuring zone, i.e. the part where the aqueous fluid part to be measured on is located when the NMR measurement is performed, is preferably relatively spatially homogeneous and relatively temporally constant. However, in general it is difficult to ensure that the magnetic field in the measuring zone is entirely homogenous, and further for most magnetic fields the field strength might drift or vary over time due to aging of the magnet, movement of metal objects near the magnet, and temperature fluctuations.

Drift and variations over time can be dealt with by controlling the temperature and/or by applying a field lock such as it is generally known in the art.

Spatial inhomogeneities of the magnetic field can be corrected by a simple calibration or alternatively or simultaneously, such spatial inhomogeneities can be adjusted by shim coils such as it is also known in the art. Such shim coils may e.g. be adjusted by the computer to maximize the homogeneity of the magnetic field.

In an embodiment of the invention, the method comprises performing a plurality of NMR measurements at a selected magnetic field, preferably the magnetic field is kept substantially stationary during the plurality of NMR measurements.

In an embodiment, the method of the invention comprises regulating the temperature e.g. by maintaining the temperature at a selected value.

In an embodiment, the method comprises performing the NMR reading at a fixed temperature.

In an embodiment, the method of the invention comprises determining the temperature.

In an embodiment, the method of the invention comprises performing the NMR readings at pulsed temperature.

In an embodiment, the method comprises performing the NMR reading at a temperature which is pulsed, preferably the pulsing range is from about 1 °C to about 90 °C, such as from about 10 °C to about 80 °C, such as from about 20 °C to about 70 °C. The pulsed temperature may advantageously be applied for correlation of resulting measurements at different temperatures to eliminate errors and/or for improved pH determination as described above.

In order to increase the accuracy of the nitrogen determination it may be advantageous to remove solid parts from the aqueous fluid - e.g. solid parts from the aqueous fluid in the form of a manure slurry.

In an embodiment, the method of the invention is applied to analyze the nutrient condition of soil on a field to thereby ensure that the amount of fertilizer added or to be added is as optimal as possible. It has been found that performing ¹⁴N NMR analysis directly on soil gives a poor result. Therefore the method for analyzing the soil advantageously comprises providing an aqueous fluid comprising soil or fragments of soil, such as farming soil and this aqueous fluid is used in the method of the invention.

Preferably, the part of the aqueous fluid is in the form of an aqueous soil extraction of a soil sample. The aqueous soil extraction is preferably obtained from washing the (preferably pre dried) soil sample with a predetermined amount of water, such as water without any inorganic ¹⁴N and/or ³¹P component contents or with a known inorganic ¹⁴N and/or ³¹P component contents, such as substantially pure water e.g. water obtained from a reverse osmosis filtration.

The soil sample is advantageously pre-dried to constant weight at 25 °C.

The washing of the soil sample with water comprises preferably mixing the soil sample with the water and subjecting the mixture to a filtration to filter out a major amount by weight of the soil, the filtration is preferably being a microfiltration (e.g. with cut off limit of about 200 Da) or a reverse osmosis filtration.

Advantageously, a plurality of soil samples of a farmer field is obtained at a preselected depth. The depth is selected in dependence on which kind of crop is to be grown on the field i.e. the depth is advantageously where the crop is expected to obtain water and nutritents. Thereby the nutrition state of the field can be mapped. The sampling from the field can e.g. be obtained simultaneously with the farmer is preparing the soil for seeding or even when he is performing the seeding. From the mapped nutrition state, the farmer can ensure an optimized fertilization, which e.g. may comprise that certain areas of the field are given a larger amount of fertilizer than other areas of the field.

In an embodiment, the method comprises performing a plurality of NMR measurements on the aqueous fluid. Advantageously the NMR measurement of the aqueous fluid will be performed a plurality of times in order to reduce the noise. In an embodiment, the NMR measurement is performed continuously in repeated measuring cycles. In an embodiment, the method comprises performing a plurality of NMR measurements on the same aqueous fluid part (e.g. a sample). The NMR measurements are normally performed very fast e.g. several NMR measurement circles per second, such as 20 NMR measurements or more, such as 50 NMR measurements or more. Therefore, even when performing the NMR measurement on the aqueous fluid part in a flowing condition, several NMR measurements may be performed on virtually the same aqueous fluid part.

In an embodiment the NMR measurement comprises simultaneously subjecting the aqueous fluid part to a magnetic field B and an exciting RF pulse with frequencies selected to excite a nuclei spin of at least a part of the ¹⁴N isotope. Preferably, the exciting RF pulse has a band width (span over a frequency range) which is sufficient to excite nuclei spin (spin transition) of substantially all ¹⁴N isotopes in the aqueous fluid part subjected to the measurement.

A general background description of NMR measurement can be found in "NMR Logging Principles and Applications" by George R. Coates et al, Halliburton Energy Services, 1999. See in particular chapter 4. Although this document does not specifically describe the NMR determination of nitrogen isotope, the principle applied is similar.

The nitrogen quadrupole splitting reflects the interaction between the nuclear energy levels and surrounding electric field gradient (EFG). Nuclei in states with non-spherical charge distributions, such as ¹⁴N isotope, produce an asymmetrical electric field, which splits the nuclear energy levels. This produces a nuclear quadrupole moment, resulting in optional splitting up of signals from an ¹⁴N isotope, dependent on its position in a compound or an ion and in particular dependent on the symmetry of a compound it is part of. The splitting up of signals from a ¹⁴N isotope is called the quadrupole broadening.

In an embodiment, the aqueous fluid part is subjected to an exciting RF pulse with frequencies selected to excite substantially all nuclei spins of substantially all ¹⁴N isotopes in the aqueous fluid part.

In an embodiment the method comprises transmitting narrow bandwidth pulses, such as with a bandwidth of from about 10 ppm to about 200 ppm, such as from about 30 ppm to about 300 ppm, to thereby stimulate only part of the nitrogen spectra for quantitative determination of relative amounts of two or more different nitrogen compounds or ions thereof, the determination is preferably performed using a substantially homogeneous magnetic field e.g. a homogenous magnet field such as from 30 ppm to about 300 ppm.

A sufficient frequency range of radio pulses (band width) can be found by performing a calibration test on aqueous fluids with a known content of nitrogen compound that is desired to be determined. The ¹⁴N isotopes in aqueous fluids will normally be excited at least in their central bands within a relatively small frequency range of radio pulses.

A chemical shift is defined as the relative difference in resonant frequency compared to a reference signal. The shift is believed to be caused by spin-spin coupling between protons of compounds. In an embodiment the method comprises measuring at least one frequency shift, preferably the measured frequency shift compared with a suitable reference (such as a only NH₄⁺ in water) of the read ¹⁴N data spectra is used for quantitative determination of relative amounts of two or more different nitrogen compounds or ions thereof preferably in combination with other NMR data.

In an embodiment, the radio frequency pulses are in the form of adiabatic RF pulses, i.e. RF pulses that are amplitude and frequency modulated pulses.

In an embodiment of the invention, the frequency range of the exciting RF pulse comprises a band width of at least about 1 MHZ.

By a few trial and error tests, the desired frequency range for at specific type of determination can be found.

The actual frequencies that are exciting the spin of the ¹⁴N isotope nucleus depend largely on the magnetic field B. As explained above, the magnetic field may vary due to drift and due to temperature variations and it is generally preferred that the exciting RF pulses are adjusted by a field lock function in order to ensure that the NMR measurements are performed using exciting RF pulses which are directed towards desired nucleus spin of the ¹⁴N isotope.

In an embodiment, the method of the invention comprises determining at least one relaxation rate of an excited ¹⁴N isotope.

In an embodiment, the ¹⁴N data comprises ¹⁴N T1 data and/or ¹⁴N T2 data.

The term relaxation describes processes by which nuclear magnetization excited to a non-equilibrium state returns to the equilibrium distribution. In other words, relaxation describes how fast spins "forget" the direction in which they are oriented. Methods of measuring relaxation times T1 and T2 are well known in the art.

In an embodiment, the method comprises determining at least one spin-lattice - T1 relaxation value of an excited ¹⁴N isotope.

It is believed that T1 relaxation involves redistributing the populations of nuclear spin states in order to reach the thermal equilibrium distribution.

T1 relaxation values may be dependent on the NMR frequency applied for exciting the ¹⁴N isotope. This should preferably be accounted for when analyzing and calibrating the T1 relaxation values obtained.

In an embodiment, the method comprises determining at least one spin-spin - T2 relaxation value of an excited ¹⁴N isotope. The T2 relaxation is also called the transverse relaxation. Generally, T2 relaxation is a complex phenomenon and involves decoherence of transverse nuclear spin magnetization. T2 relaxation values are substantially not dependent on the magnetic field applied during excitation of the ¹⁴N isotope, and for most determinations such possible variations can be ignored.

In an embodiment the method comprises subjecting the aqueous fluid part to pulsed trains of RF pulses, preferably with repetition rates of at about 100 ms or less, such as from about 10 to about 50 ms, such as from about 15 to about 20 ms.

The trains of RF pulses are often applied to determine the T1 and/or T2 values.

In an embodiment, the method comprises subjecting the aqueous fluid part to trains of square RF pulses, preferably with repetition rates of about 100 ms or less, such as about 10 ms or less, such as about 5 ms or less, such as about 1 ms or less.

A short square pulse of a given "carrier" frequency "contains" a range of frequencies centered about the carrier frequency, with the range of excitation (bandwidth/ frequency spectrum) being inversely proportional to the pulse duration.

A Fourier transform of an approximately square wave contains contributions from all the frequencies in the neighborhood of the principal frequency. The restricted range of the NMR frequencies made it relatively easy to use short (millisecond to microsecond) radio frequency pulses to excite the entire NMR spectrum.

In an embodiment, the NMR measurement comprises simultaneously subjecting the aqueous fluid part to a magnetic field B and a plurality of RF pulses wherein the RF pulses comprise
i. an exciting RF pulse , and
ii. at least one refocusing RF pulse.

The exciting RF pulse and the refocusing pulse or pulses may for example be in the form of a train of RF pulses, e.g. pulsed pulses. The exciting RF pulse is preferably as described above and may in an embodiment be pulsed.

Useful duration and amplitude of the exciting RF pulses are well known in the art and optimization can be done by a simple trial and error.

In an embodiment, the exciting RF pulse is in the form of a 90° pulse.

A 90° pulse is an RF pulse designed to rotate the net magnetization vector 90° from its initial direction in the rotating frame of reference. If the spins are initially aligned with the static magnetic field, this pulse produces transverse magnetization and free induction decay (FID).

In an embodiment the refocusing RF pulse(s) is in the form of a 180° pulse, preferably the method comprises subjecting the aqueous fluid part to a plurality of refocusing RF pulses, such as one or more trains of refocusing RF pulses.

A 90° pulse is an RF pulse designed to rotate the net magnetization vector 180° in the rotating frame of reference. Ideally, the amplitude of a 180° pulse multiplied by its duration is twice the amplitude of a 90° pulse multiplied by its duration. Each 180° pulse in the sequence (called a CPMG sequence after Carr-Purcell-Meiboom-Gill) creates an echo.

A standard technique for measuring the spin-spin relaxation time T2 utilizing CPMG sequence is as follows. As is well known after a wait time that precedes each pulse sequence, a 90-degree exciting pulse is emitted by an RF antenna, which causes the spins to start processing in the transverse plane. After a delay, an initial 180-degree pulse is emitted by the RF antenna. The initial 180-degree pulse causes the spins, which are dephasing in the transverse plane, to reverse direction and to refocus and subsequently cause an initial spin echo to appear. A second 180-degree refocusing pulse can be emitted by the RF antenna, which subsequently causes a second spin echo to appear. Thereafter, the RF antenna emits a series of 180-degree pulses separated by a short time delay. This series of 180-degree pulses repeatedly reverse the spins, causing a series of "spin echoes" to appear. The train of spin echoes is measured and processed to determine the spin-spin relaxation time T2.

In an embodiment, the refocusing RF pulse(s) is/are applied with an echo-delay time after the exciting RF pulse. The echo-delay time (also called wait time TW) is preferably of about 500 µs or less, more preferably about 150 µs or less, such as in the range from about 50 µs to about 100 µs.

This method is generally called the "spin echo" method and was first described by Erwin Hahn in 1950. Further information can be found in Hahn, E.L. (1950). "Spin echoes". Physical Review 80: 580-594.

A typical echo-delay time is from about 10 µs to about 50 ms, preferably from about 50 µs to about 200 µs. The echo-delay time (also called wait time TW) is the time between the last CPMG 180° pulse and the first CPMG pulse of the next experiment at the same frequency. This time is the time during which magnetic polarization or T1 recovery takes place. It is also known as polarization time.

This basic spin echo method provides very good result for obtaining T1 relaxation values by varying TW and T2 relaxation values can also be obtained by using plurality of refocusing pulses.

The refocusing delay is also called the Echo Spacing and indicates the time identical to the time between adjacent echoes. In a CPMG sequence, the TE is also the time between 180° pulses.

This method is an improvement of the spin echo method by Hahn. This method was provided by Carr and Purcell and provides an improved determination of the T2 relaxation values.

Further information about the Carr and Purcell method can be found in Carr, H. Y.; Purcell, E. M. (1954). "Effects of Diffusion on Free Precession in Nuclear Magnetic Resonance Experiments". Physical Review 94: 630-638.

There are several types of NMR experiments that depend on the introduction of a second irradiation frequency, i.e. irradiation of a nucleus other than the one being observed. In an embodiment the method of the invention comprises introduction of a second irradiation frequency irradiating of a nucleus other than the one being observed, such as ¹⁴N.

In an embodiment the method comprising enhancing signal to noise of the data spectra by subjecting the aqueous liquid to polarization treatment during the NMR reading, the polarization treatment preferably comprises DEPT (Distortionless Enhancement by Polarization Transfer) or NOE (Nuclear Overhauser Effect).

DEPT has heretofore been applied as a very useful method for determining the presence of primary, secondary and tertiary carbon atoms using ¹³C NMR. The DEPT experiment differentiates between CH, CH₂ and CH₃ groups by variation of the selection angle parameter. It has been found that the DEPT method used in combination with ¹⁴N can provide a similar enhancement.

The Nuclear Overhauser Effect (NOE) is the transfer of nuclear spin polarization from one nuclear spin population to another via cross-relaxation. It is a common phenomenon observed by nuclear magnetic resonance (NMR) spectroscopy. Nuclear Overhauser Effect can for example be used to determine intra- (and even inter-) molecular distances. The NOE effect is the change in population of one proton (or other nucleus) when another magnetic nucleus close in space is saturated by decoupling or by a selective 180 degree pulse.

Further information about DEPT and NOE and how to perform them can e.g. be found in "New Method for NMR Signal Enhancement by Polarization Transfer, and Attached Nucleus Testing" by John Homer et al,. J. Chem. Soc.,Chem. Commun., 1994 and "Fundamentals of NMR" Chapter 1, by Thomas L. James, Department of Pharmaceutical Chemistry, University of California, 1998 (http://qudev.ethz.ch/content/courses/phys4/studentspresentations/nmr/James _Fundamentals_of_NMR.pdf)

In an embodiment the polarization treatment comprises subjecting the aqueous liquid to cross polarization to thereby enhance quantitative determination of relative amounts of two or more different nitrogen compounds or ions thereof, the cross polarization is preferably in the form of DEPT.

In an embodiment, the method comprises subjecting a sample of the aqueous fluid to the NMR reading comprising circulating the sample and generating ¹⁴N data pool from a plurality of consecutive NMR readings comprising a ¹⁴N NMR data spectra and correlating the ¹⁴N NMR data pool to the calibration data.

In an embodiment, the method comprises providing calibration data of aqueous fluids with known concentrations of nitrogen components. The calibration data constitutes a calibration map. The calibration map comprises the desired NMR data and optionally additionally data such as data relating to temperature(s), pH value(s) and or relative amounts of selected components in dependence of pH value and/or temperature - e.g. as the relation shown in Fig. 1.

The term 'calibrating map' is herein used to designate a collection of NMR spectra data obtained in aqueous fluids with known amounts of nitrogen containing compounds and optionally other data which can be used in the interpretation of NMR data. The calibration map may be in the form of raw data, in the form of drawings, in the form of graphs, in the form of formulas or any combinations thereof.

In an embodiment, the aqueous fluid used for generating the calibrating map is of a similar type as the aqueous fluid to be tested. In an embodiment, the calibration map also comprises a plurality of values determined on an aqueous fluid mixed with additional water.

Generally, it is well known in the art to calibrate NMR measurements based on NMR spectra obtained on known compositions.

In an embodiment, the calibration map is in the form of a pre-processed data set, where the NMR spectra obtained for an aqueous fluid under analysis can be processed by the computer to provide a clear level, amount or concentration of nitrogen containing compounds in the aqueous fluid.

In an embodiment, the method comprises preparing calibration data and storing the calibration data on a digital memory. The method advantageously comprises feeding the NMR spectrum(s) or data obtained from the NMR spectrum(s) to a computer in digital communication with the digital memory and providing the computer to compare and analyze the data to perform at least one quantitative determination of one or more nitrogen containing components.

The calibration map may be built up during use, for example additional data obtained by measurement on the aqueous fluid is fed to the computer and used in the calibration of the data for later determinations.

The computer may for example be programmed to compute the data obtained using artificial intelligence or the calibration map may be applied to teach a neural network.

In an embodiment the method comprises performing at least one NMR measurement on a plurality of aqueous fluid parts, preferably the method comprises performing a plurality of NMR measurements and optionally other measurements, such as 1H NMR measurement, ¹³C NMR measurements, ³¹P NMR measurements and/or ³⁹K NMR measurements.

In order to improve the determination in the aqueous fluid, other compounds can additionally and preferably simultaneously be determined in the aqueous fluid.

In an embodiment the method further comprises quantitative determination of at least one carbon containing component, preferably by subjecting at least a part of the aqueous fluid to an NMR reading comprising generating a ¹³C data and correlating the ¹³C NMR data to calibration data. The NMR measurement preferably comprises obtaining at least one spin-lattice - T1 value and at least one spin-spin - T2 value of an excited ¹³C isotope.

The ¹³C determination using NMR can be performed in a similar manner as the method described above but by using other frequencies and optionally the strength of the magnetic field may also be adjusted. The skilled person will know how to perform such determinations. In an embodiment the determination of carbon is performed using the same hardware (magnet, pulse emitter, receiver and similar) as used in the nitrogen ¹⁴N determination. Thereby the equipment and the setup is economical feasible.

In an embodiment the method further comprising quantitative determination of hydrocarbon containing components, preferably by subjecting at least a part of the aqueous fluid to an NMR reading in combination with cross polarization preferably in the form of DEPT comprising generating a ¹³C data and correlating the ¹³C NMR data to calibration data.

In an embodiment the method further comprising performing a quantitative estimation of organic nitrogen compounds having a molecular weight of at least about 200 Da in the aqueous fluid by assuming a fixed quantitative relationship of ¹³C (or hydrocarbons components) to such organic nitrogen compounds.

In the following is described a system suitable for quantitative determination of nitrogen in an aqueous fluid as described above.

The system comprises a NMR spectrometer configured for obtaining ¹⁴N NMR spectra, a digital memory storing a calibration map comprising calibrating data for calibrating ¹⁴N NMR spectra obtained by the NMR spectrometer and a computer programmed to analyze the ¹⁴N NMR spectra obtained by the NMR spectrometer using the calibration map and performing at least one quantitative nitrogen determination.

The spectrometer may be any NMR spectrometer suitable for use in performing the method of the invention. Advantageously, the spectrometer is as described above and should preferably be configured to perform a NMR reading of an aqueous fluid. Preferably, the magnet(s) of the spectrometer is as described above. Advantageously the NMR spectrometer comprises one or magnets arranged to generate a substantially magnetic field of from 0.1 to 2 tesla. The calibration map may be as described above.

The calibration map may be continuously updated with new data.

The system may comprise one, two or more computers, one, two or more spectrometers and/or one, two or more calibration maps. The system may preferably be in data communication with the internet e.g. for communication with other similar systems, for sending and/or receiving data. The system may preferably comprise at least one display and/or an operating keyboard as well as any other digital equipment usually connected to digital systems, e.g. printers.

The system may further comprises a digital memory storing a calibration map for one or more of the isotopes ³⁹K isotope, ³¹P isotope and ¹³C isotope, the map comprises calibration data for said one or more isotopes and optionally of amounts thereof in petroleum fuels.

The system is advantageously configured to perform NMR measurement on an aqueous fluid in a flowing condition.

All features of the inventions including ranges and preferred ranges can be combined in various ways within the scope of the appended claims, unless there are specific reasons not to combine such features.

### BRIEF DESCRIPTION OF EXAMPLES AND DRAWINGS

The invention will be explained more fully below in connection with illustrative examples and embodiment and with reference to the drawings in which:
FIG. 1 shows a diagram over the relationships between NH₃ and NH4⁺ in dependence on temperature and pH value.
FIG. 2 is a schematic drawing of a system for quantitative determination of nitrogen in aqueous fluid.
FIG. 3 is a schematic side view of a movable manure spreader which may be applied for spreading manure tested using the method of the invention.
FIG. 4 is a schematic top view of a farmer field where coordinates are marked indicating where soil samples are taken for analyzing for nutrients.
FIG. 5 shows a part of an exemplified farmer field map (table 1) of nitrogen content and optionally phosphor and/or potassium map.
FIGs. 6a and 6b shows a table 2 over reference determinations of Ammonium and Potassium.
FIG. 7 shows the mean of NMR determinations of Ammonium and Potassium versus the individual results of NMR determinations of Ammonium and Potassium.
FIG. 8 shows the mean of lab determinations versus the mean of the NMR determinations of Ammonium and Potassium.
FIG. 9 shows the correlation between amount of dry matter and organic nitrogen.

The diagram of Fig. 1 shows the relationships between NH₃ and NH4⁺ in dependence on temperature and pH value.

As mentioned the relative amount of NH₃ versus NH4⁺ is a very important knowledge for example for determination of the pH value in liquid manure. Daily or even hourly determinations of the amount of NH₃ versus NH4⁺ can for example be used to determine the health of the animals from which the manure is originating. Further, such determination can be used to predict how long the farmer can store the liquid manure. Advantageously, the content of this diagram is included in the calibration map.

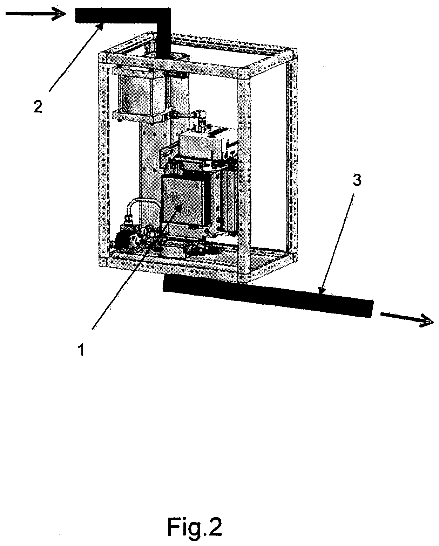

FIG. 2 is a schematic drawing of a system of the invention for quantitative determination of nitrogen in aqueous fluid.

The system is suitable for quantitative determination of nitrogen present in the form of one or more nitrogen containing compounds or ions thereof in an aqueous fluid. The system comprises a NMR spectrometer 1 configured for obtaining ¹⁴N NMR spectra, a not shown digital memory storing a calibration map comprising calibrating data for calibrating ¹⁴N NMR spectra obtained by the NMR spectrometer and a not shown computer programmed to analyze the ¹⁴N NMR spectra obtained by the NMR spectrometer using the calibration map and performing at least one quantitative nitrogen determination. The system comprises an in-flow pipe 2 and an out-flow pipe 3 for the aqueous fluid. The system is configured to perform ¹⁴N NMR measurement on an aqueous sample in a flowing condition.

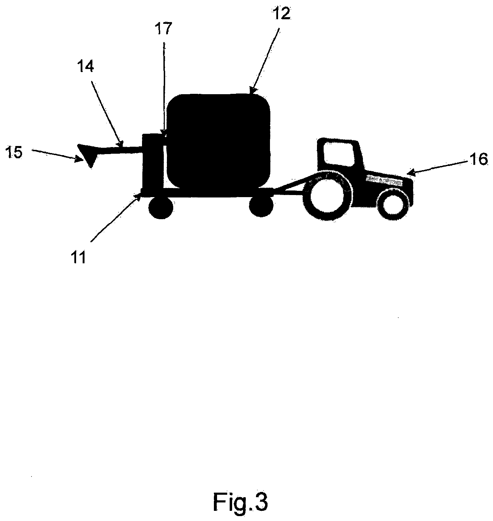

The movable manure spreader shown in Fig. 3 comprises a movable platform truck 11 carrying a tank 12 for containing liquid manure. The platform truck 11 further carries a system 13 comprising a NMR spectrometer and being suitable for quantitative determination of nitrogen present in the form of one or more nitrogen containing compounds or ions thereof in the liquid manure. The system 13 may be as described above. The system 13 comprising the NMR spectrometer is in fluid connection with the tank 12 via a pipe. The system 13 comprises an additional fluid connection, which here is a pipe 14 with a spreader 15 for spreading the liquid manure on a farmer field. A tractor 16 is connected to the platform truck 11.

The liquid manure passes from the tank 12 via the pipe 17 and into the system 13 comprising the NMR spectrometer wherein the content of nitrogen present in the form of one or more nitrogen containing compounds or ions thereof in the liquid manure is determined by the method of an embodiment of the invention. The system 13 advantageously is also configured to determine the content of phosphor and/or potassium. The liquid manure flows further via the pipe 14 to the spreader 15 by which it is spread onto a not shown farmer field as the platform truck 11 is pulled forward by the tractor 16.

The fluid connection which in the shown example is a pipe 14 with a spreader 15 can for example be replaced with an inflow pipe connecting a liquid manure reservoir to the tank 12 via the system 13 comprising the NMR spectrometer. Thereby the content of nitrogen present in the form of one or more nitrogen containing compounds or ions thereof in the liquid manure can be determined in the system 13 during filling of the tank 12 with the liquid manure and the average content of the nitrogen content can for example be determined for example together with a determination of the homogeneity/inhomogeneity of the nitrogen content of the liquid manure. Simultaneously the content of phosphor and/or potassium can be determined.

The system 13 optionally comprises a filter as described above.

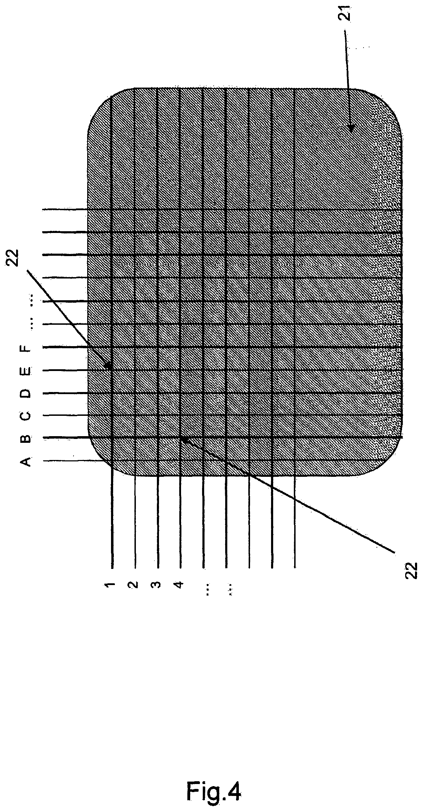

The farmer field shown in Fig. 4 has been marked with coordinates to indicate where soil samples are taken for analyzing the content of one or more nutrients. Soil samples are taken at a selected depth or several selected depths in each cross-point 22. The soil samples may e.g. be taken with a minimum distance of about 5 to about 100 m to each other.

The soil samples are advantageously pre-dried and an washed with water to obtain a water extract of each soil sample e.g. as described above and the content of nitrogen present in the form of one or more nitrogen containing compounds or ions thereof in the extract is determined by the method as described herein e.g. using the system shown in Fig. 2. Simultaneously the content of phosphor and/or potassium can be determined.

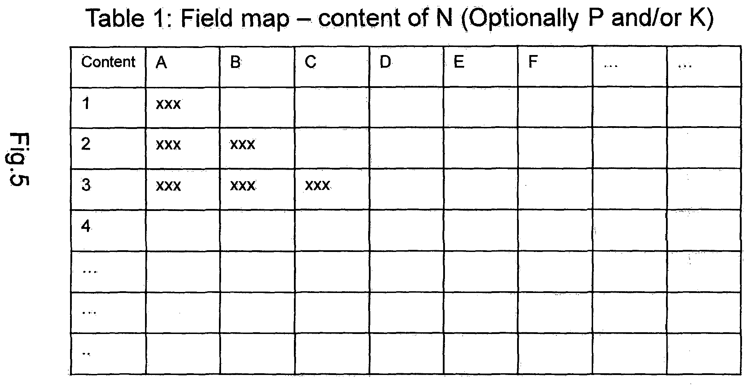

Thereafter the nutrition state of the field can be mapped e.g. as shown in the farmer field map of nitrogen content and optionally phosphor and/ potassium map of Fig. 5 in form of a table (table 1)where the 'xxx' indicates different results of nutrition contents determined in each soil sample.

The farmer field map can e.g. be loaded into a computer or connected to the system 13 comprising the NMR spectrometer shown in Fig. 3 and the amount of liquid manure spread via the spreader 15 is controlled in dependence of the nutrition state determined at the cross-points and the nitrogen (and optionally of phosphor and/or potassium content) and the determined content of such nutrition's determined in the liquid manure. Thereby the fertilization can be optimized.

### Example 1

¹⁴N NMR data spectra are obtained from water samples with known amounts of Nitrite (NO₂⁻), Nitrate (NO₃⁻), Melamine (C₃H6N6), Urea ((NH₂)₂CO), NH₃, NH₄⁺. At least 5 water samples of each nitrogen component with different amount are analyzed and each sample is subjected to a plurality of tests cycles to eliminate or reduce errors. The ¹⁴N NMR data spectra comprises T1 and T2 data, data obtained by DEPT and/or NOE as well as data obtained at different temperatures. The obtained ¹⁴N NMR data spectra are collected in a calibration map together with the diagram shown in Fig. 1.

### Example 2

Liquid manure from a livestock of pigs is filtered using a 1 mm masked filter to remove coarse particles. The filtrated liquid pig manure is subjected to an ¹⁴N NMR analysis in a system of the invention comprising the calibration map of Example 1 in its memory. The ¹⁴N NMR data obtained from the analysis of the liquid pig manure is correlated with the calibration map and the content of nitrogen present in the form of at least one of the nitrogen containing compounds Nitrite (NO₂⁻), Nitrate (NO₃⁻), Melamine (C₃H6N6), Urea (NH₂)₂CO), NH₃, NH₄⁺ is determined.

### Example 3

A system comprising a NMR spectrometer configured to obtain ¹⁴N NMR spectra was provided together with a digital memory storing a calibration map comprising calibrating data for calibrating ¹⁴N NMR spectra obtained by the NMR spectrometer and a computer programmed to analyze the ¹⁴N NMR spectra obtained by the NMR spectrometer.

Further, a master sample of 20 different slurries and solutions was provided.

From each master sample two samples was withdrawn and the samples was randomly numbered from 1-40. Samples Nos. 38, 40, 19 and 20 was diluted with tap water as shown in the Fig. 2 samples. The samples was used for tests in the NMR spectrometer, the volume of the samples for test in the NMR spectrometer was about 25ml each.

The test in the NMR spectrometer was performed at a substantially homogeneous field of about 1.5 Tesla. The ¹⁴N NMR data spectra comprised T1 and T2 data, data obtained by DEPT and/or NOE. Further ¹⁷O, ³¹P and ³⁹K NMR data was obtained. The NMR reading time for each sample was 30 minutes.

Base on the obtained NMR data total amount of N (g/l), total amount of K (g/l) and the amount of ammonium was determined for the individual samples.

Further each sample was tested for Dry matter (%). Dry matter was determined based on the ¹⁷O NKR readings and as described in DK-PA 2014 70339.

The tests were repeated 5 times for each sample and the mean value for each sample was determined.

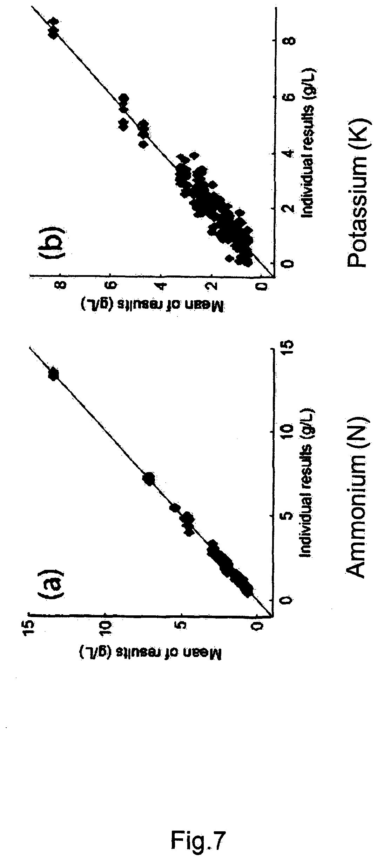

The mean value of Ammonium amount and Potassium amounts respectively for each sample was plotted versus the individual results of the NMR determinations for the respective samples. The results are shown in Fig. 7. It can be seen that the deviation between the individual samples are very small which is a clear indication that the method of the invention gives very reliable results.

### Example 4

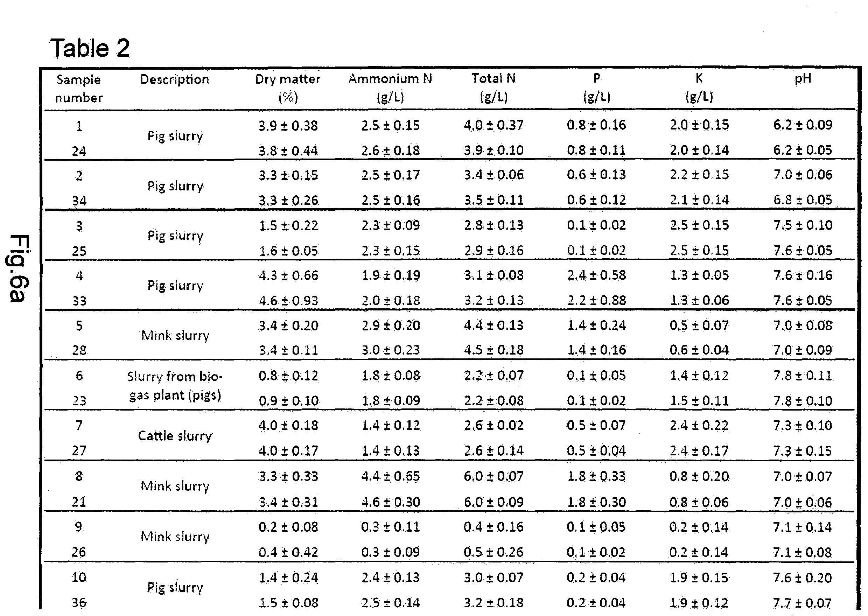

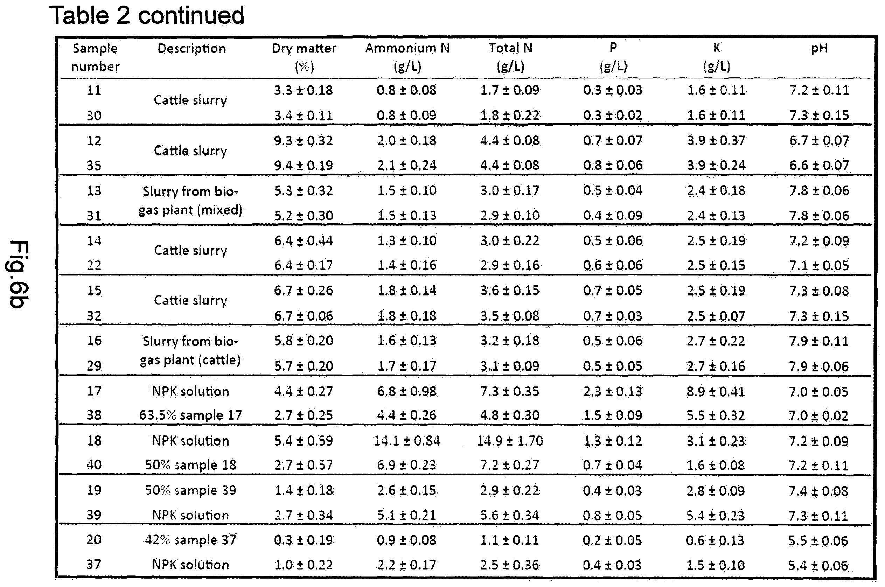

From each master sample of example 3, two samples was withdrawn and numbered as in example 3 and the samples Nos. 38, 40, 19 and 20 was diluted with tap water as shown in the Fig. 2 The samples was randomly divided into 5 groups and sent to 5 different independent laboratories for test for Dry matter (%), Ammonium N (g/L), Total N (g/L), P N (g/L), K N (g/L) and pH. The results are shown in table 2, Figs 6a, 6b. As shown in the table 2, the samples 1 and 24 are identical, samples 2 and 34 are identical and etc. The laboratory test was use as reference test.

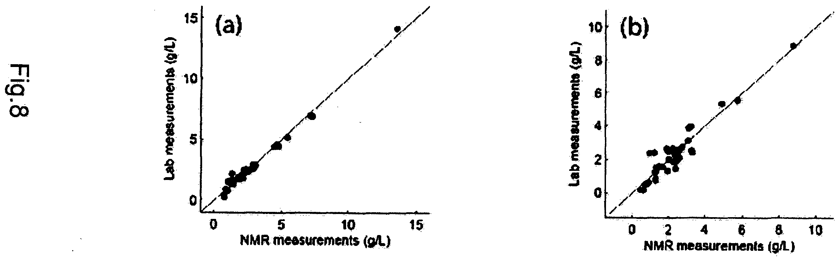

The mean value of the respective laboratory determinations versus the mean value of the NMR determinations for respective N and K were plotted. The results are shown in Fig. 8. It can be seen that the results obtained by NMR determinations are very similar to the results obtained by the laboratory tests.

### Example 5

From each master sample of example 3, two samples was withdrawn and numbered as in example 3 and the samples Nos. 38, 40, 19 and 20 was diluted with tap water as shown in the Fig. 2

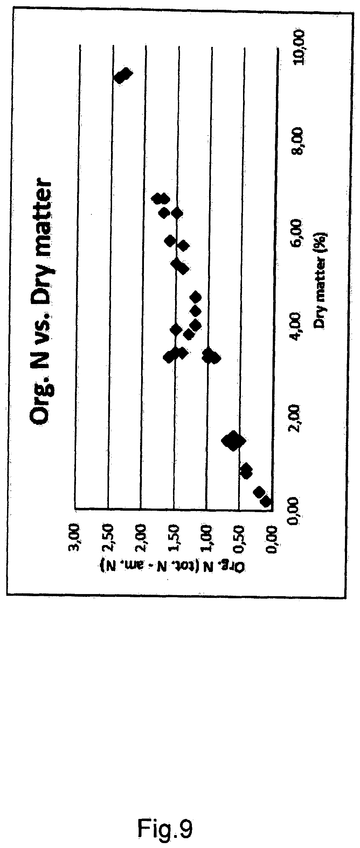

The amount of organically bound nitrogen was determined in each of the sample. The result was plotted versus the dry matter percentage determined in example 4 and the result is shown in Fig. 9. It can be seen that there is a good overall correlation between amount of dry matter and amount of organically bound nitrogen in the slurries. From this, it can be concluded that the amount of organically bound nitrogen in slurries can be estimated with a high certainty from the amount of dry matter.

## Claims

1. A method for quantitative determination of nitrogen present in the form of one or more nitrogen containing compounds and/or ions thereof in an aqueous fluid, the method comprising subjecting at least a part of the aqueous fluid to a nuclear magnetic resonance (NMR) reading comprising generating a ¹⁴N data comprising a ¹⁴N NMR data spectra and correlating the ¹⁴N NMR data to calibration data, **characterized in that** no reference components are added and the aqueous fluid is substantially free of deuterium oxide and/or acetonitrile and wherein the aqueous fluid is lake or sea water, process wastewater, non-treated wastewater, treated wastewater, ground water or tap water or wherein the aqueous fluid comprises a mammal body fluid or an aqueous soil extraction of a soil sample, preferably the method comprises quantitative determination of one or more nitrogen containing compounds or ions thereof, more preferably the method comprises determination of the content of nitrogen containing compounds or ions thereof in said aqueous fluid.

2. The method of claim 1 wherein the one or more nitrogen containing compounds are dissolved or dispersed in the aqueous fluid, preferably the one or more nitrogen containing compounds are dissolved in the aqueous fluid and have a molecular weight of up to about 200 Da, such as from about 15 to about 100 Da.

3. The method of any one of the preceding claims wherein the NMR reading is performed on the aqueous fluid in a flowing condition or in a semi-flowing condition, preferably the NMR reading is performed in an inline process during the transportation of the aqueous fluid from one fluid reservoir to another and the method further comprises determination of the flow of the aqueous fluid in the magnet field.

4. The method of any one of the preceding claims wherein the method comprises quantitative determination of Nitrite (NO₂⁻), Nitrate (NO₃⁻), Melamine (C₃H₆N₆), Urea ((NH₂)₂CO), NH₃, NH₄⁺, or any combinations thereof.

5. The method of any one of the preceding claims wherein the method comprises determination of the relative amounts of Nitrate (NO₃⁻) versus ammonia (NH₃ and NH₄⁺) and/or the method comprises determination of the relative amounts of NH₃ versus NH4⁺.

6. The method of any one of the preceding claims wherein the aqueous fluid is or comprises a mammal body fluid selected from urine and/or is liquid manure.

7. The method of any one of the preceding claims wherein the NMR reading is performed in a magnetic field of up to about 2.5 Tesla.

8. The method of any one of the preceding claims wherein the aqueous fluid is in the form of the aqueous soil extraction of a soil sample, the aqueous soil extraction is obtained from washing the soil sample with a predetermined amount of water, such as water with a known inorganic ¹⁴N and/or ³¹P component contents, such as substantially pure water e.g. water obtained from a reverse osmosis filtration.

9. The method of claim 8 wherein the washing of the soil sample with water comprises mixing the soil sample with the water and subjecting the mixture to a filtration to filter out a major amount by weight of the soil, the filtration is preferably being a microfiltration e.g. with cut off limit of about 200 Da or a reverse osmosis filtration.

10. The method of any one of the preceding claims wherein the method comprising enhancing signal to noise of the data spectra by subjecting the aqueous liquid to polarization treatment during the NMR reading, the polarization treatment preferably comprises DEPT (Distortionless Enhancement by Polarization Transfer) or NOE (Nuclear Overhauser Effect).

11. The method of any one of the preceding claims wherein the method further comprises quantitative determination of at least one phosphor containing component and/or quantitative determination of at least one inorganic potassium containing ion.

12. The method of any one of the preceding claims wherein the method further comprises quantitative determination of at least one carbon containing component, preferably by subjecting at least a part of the aqueous fluid to an and/or quantitative determination of hydrocarbon containing components, preferably by subjecting at least a part of the aqueous fluid to an NMR reading in combination with cross polarization preferably in the form of DEPT comprising generating a ¹³C data and correlating the ¹³C NMR data to calibration data, preferably the method further comprises performing a quantitative estimation of organic nitrogen compounds having a molecular weight of at least about 200 Da in the aqueous fluid by assuming a fixed quantitative relationship of ¹³C or hydrocarbons components to such organic nitrogen compounds.

13. The method of any one of the preceding claims wherein the method further comprises determining the amount of H₂O in the aqueous fluid and thereby determining the dry matter amount, the amount of H₂O is preferably determined by performing at least one NMR reading of ¹⁷O nuclei in the aqueous fluid, the reading comprises obtaining ¹⁷O NMR data and determining the amount of H₂O in a sample by correlating the ¹⁷O NMR data to calibration data, preferably the content of organically bound nitrogen is estimated based on the content of dry matter.

14. The method of any one of the preceding claims wherein the actually measured temperature of at least part of the aqueous fluid and the relatively measured contents of NH₃ versus NH4⁺ are used to calculate and display pH value in the range 5.5 to 12.

## Patentansprüche

1. Verfahren zur quantitativen Bestimmung von Stickstoff, der in Form einer oder mehrerer stickstoffhaltiger Verbindungen und/oder Ionen davon in einer wässrigen Flüssigkeit vorhanden ist, wobei das Verfahren das Unterziehen mindestens eines Teils der wässrigen Flüssigkeit einer kemmagnetischen Resonanz (NMR)-Messung umfasst, die das Erzeugen von ¹⁴N-Daten, die ein ¹⁴N-NMR-Datenspektrum umfassen, und das Korrelieren der ¹⁴N-NMR-Daten mit Kalibrierungsdaten umfasst, **dadurch gekennzeichnet, dass** keine Referenzkomponenten zugesetzt werden und die wässrige Flüssigkeit im Wesentlichen frei von Deuteriumoxid und/oder Acetonitril ist und wobei die wässrige Flüssigkeit See- oder Meerwasser ist, Prozessabwasser, unbehandeltes Abwasser, behandeltes Abwasser, Grundwasser oder Leitungswasser ist oder wobei die wässrige Flüssigkeit eine Säugetierkörperflüssigkeit oder eine wässrige Bodenextraktion einer Bodenprobe umfasst, wobei das Verfahren vorzugsweise die quantitative Bestimmung einer oder mehrerer stickstoffhaltiger Verbindungen oder Ionen davon umfasst, wobei das Verfahren vorzugsweise die Bestimmung des Gehalts an stickstoffhaltigen Verbindungen oder Ionen davon in der wässrigen Flüssigkeit umfasst.

2. Verfahren nach Anspruch 1, wobei die eine oder die mehreren stickstoffhaltigen Verbindungen in der wässrigen Flüssigkeit gelöst oder dispergiert sind, vorzugsweise die eine oder die mehreren stickstoffhaltigen Verbindungen in der wässrigen Flüssigkeit gelöst sind und ein Molekulargewicht von bis zu etwa 200 Da, wie von etwa 15 bis etwa 100 Da, aufweisen.

3. Verfahren nach einem der voranstehenden Ansprüche, wobei die NMR-Messung an der wässrigen Flüssigkeit in einem fließenden Zustand oder in einem halbfließenden Zustand durchgeführt wird, vorzugsweise wird die NMR-Messung in einem Inline-Prozess während des Transports der wässrigen Flüssigkeit von einem Flüssigkeitsreservoir zu einem anderen durchgeführt, und das Verfahren umfasst ferner die Bestimmung des Flusses der wässrigen Flüssigkeit in dem Magnetfeld.

4. Verfahren nach einem der voranstehenden Ansprüche, wobei das Verfahren die quantitative Bestimmung von Nitrit (NO₂⁻), Nitrat (NO₃⁻), Melamin (C₃H₆N₆), Harnstoff ((NH₂)₂CO), NH₃, NH₄⁺ oder beliebigen Kombinationen davon umfasst.

5. Verfahren nach einem der voranstehenden Ansprüche, wobei das Verfahren die Bestimmung der relativen Mengen von Nitrat (NO₃⁻) gegenüber Ammoniak (NH₃ und NH₄⁺) umfasst und/oder das Verfahren die Bestimmung der relativen Mengen von NH₃ gegenüber NH₄⁺ umfasst.

6. Verfahren nach einem der voranstehenden Ansprüche, wobei die wässrige Flüssigkeit eine Säugetierkörperflüssigkeit ist oder umfasst, die aus Urin ausgewählt ist und/oder flüssige Gülle ist.

7. Verfahren nach einem der voranstehenden Ansprüche, wobei die NMR-Messung in einem Magnetfeld von bis zu etwa 2,5 Tesla durchgeführt wird.

8. Verfahren nach einem der voranstehenden Ansprüche, wobei die wässrige Flüssigkeit in Form der wässrigen Bodenextraktion einer Bodenprobe vorliegt, wobei die wässrige Bodenextraktion durch Waschen der Bodenprobe mit einer vorbestimmten Menge Wasser, wie Wasser mit einem bekannten Gehalt an anorganischen ¹⁴N- und/oder ³¹P-Komponenten, wie im Wesentlichen reinem Wasser, z. B. aus einer Umkehrosmosefiltration erhaltenem Wasser, erhalten wird.

9. Verfahren nach Anspruch 8, wobei das Waschen der Bodenprobe mit Wasser das Mischen der Bodenprobe mit dem Wasser und das Unterziehen des Gemischs einer Filtration umfasst, um eine größere Gewichtsmenge des Bodens herauszufiltern, wobei die Filtration vorzugsweise eine Mikrofiltration, z. B. mit einer Trenngrenze von etwa 200 Da, oder eine Umkehrosmosefiltration ist.

10. Verfahren nach einem der voranstehenden Ansprüche, wobei das Verfahren die Verbesserung des Signal-Rausch-Verhältnisses der Datenspektren umfasst, indem die wässrige Flüssigkeit während der NMR-Messung einer Polarisationsbehandlung unterzogen wird, wobei die Polarisationsbehandlung vorzugsweise DEPT (Verzerrungsfreie Verstärkung durch Polarisationstransfer) oder NOE (Kern-Overhauser-Effekt) umfasst.

11. Verfahren nach einem der voranstehenden Ansprüche, wobei das Verfahren ferner die quantitative Bestimmung von mindestens einer phosphorhaltigen Komponente und/oder die quantitative Bestimmung von mindestens einem anorganischen kaliumhaltigen Ion umfasst.

12. Verfahren nach einem der voranstehenden Ansprüche, wobei das Verfahren ferner die quantitative Bestimmung mindestens einer kohlenstoffhaltigen Komponente umfasst, vorzugsweise durch Unterziehen mindestens eines Teils der wässrigen Flüssigkeit einer Bestimmung und/oder quantitativen Bestimmung von kohlenwasserstoffhaltigen Komponenten, vorzugsweise durch Unterziehen mindestens eines Teils der wässrigen Flüssigkeit einer NMR-Messung in Kombination mit Kreuzpolarisation, vorzugsweise in Form von DEPT, umfassend die Erzeugung von ¹³C-Daten und das Korrelieren der ¹³C-NMR-Daten mit Kalibrierungsdaten, wobei das Verfahren vorzugsweise ferner die Durchführung einer quantitativen Schätzung von organischen Stickstoffverbindungen mit einem Molekulargewicht von mindestens etwa 200 Da in der wässrigen Flüssigkeit durch Annahme eines festen quantitativen Verhältnisses von ¹³C- oder Kohlenwasserstoffkomponenten zu solchen organischen Stickstoffverbindungen umfasst.

13. Verfahren nach einem der voranstehenden Ansprüche, wobei das Verfahren ferner die Bestimmung der H₂O-Menge in der wässrigen Flüssigkeit und damit die Bestimmung des Trockenmassegehalts umfasst, wobei die H₂O-Menge vorzugsweise durch Durchführung mindestens einer NMR-Messung von ¹⁷O-Kernen in der wässrigen Flüssigkeit bestimmt wird, wobei die Messung den Erhalt von ¹⁷O-NMR-Daten und die Bestimmung der H₂O-Menge in einer Probe durch Korrelation der ¹⁷O-NMR-Daten mit Kalibrierungsdaten umfasst, wobei der Gehalt an organisch gebundenem Stickstoff vorzugsweise auf der Grundlage des Trockenmassegehalts geschätzt wird.

14. Verfahren nach einem der voranstehenden Ansprüche, wobei die tatsächlich gemessene Temperatur mindestens eines Teils der wässrigen Flüssigkeit und die relativ gemessenen Gehalte an NH₃ gegenüber NH₄⁺ verwendet werden, um den pH-Wert im Bereich von 5,5 bis 12 zu berechnen und anzuzeigen.

## Revendications

1. Procédé pour la détermination quantitative de l'azote présent sous la forme d'un ou de plusieurs composés azotés et/ou ions de tels composés dans un liquide aqueux, le procédé comprenant la soumission d'au moins une partie du liquide aqueux à une lecture de résonance magnétique nucléaire (RMN) comprenant la génération de données ¹⁴N comprenant un spectre de données de RMN-¹⁴N, et la corrélation des données de RMN-¹⁴N avec des données d'étalonnage, **caractérisé en ce qu'**aucun composant de référence n'est ajouté et le liquide aqueux est sensiblement exempt d'oxyde de deutérium et/ou d'acétonitrile et le liquide aqueux étant de l'eau de mer ou de lac, de l'eau résiduaire de processus, de l'eau résiduaire non traitée, de l'eau résiduaire traitée, de l'eau souterraine ou de l'eau de distribution ou le liquide aqueux comprenant un liquide corporel mammalien ou un extrait aqueux de sol d'un échantillon de sol, de préférence le procédé comprend la détermination quantitative d'un ou de plusieurs composés azotés ou ions de tels composés, plus préférablement le procédé comprend la détermination de la teneur en composés azotés ou ions de tels composés dudit liquide aqueux.

2. Procédé selon la revendication 1, dans lequel lesdits un ou plusieurs composés azotés sont dissous ou dispersés dans le liquide aqueux, de préférence lesdits un ou plusieurs composés azotés sont dissous dans le liquide aqueux et ont une masse moléculaire de jusqu'à environ 200 Da, comme d'environ 15 à environ 100 Da.

3. Procédé selon l'une quelconque des revendications précédentes, dans lequel la lecture de RMN est effectuée sur le liquide aqueux en un état d'écoulement ou en un état de semi-écoulement, de préférence la lecture de RMN est effectuée dans un processus en ligne au cours du transport du liquide aqueux d'un réservoir de liquide à un autre et le procédé comprend en outre la détermination de l'écoulement du liquide aqueux dans le champ magnétique.

4. Procédé selon l'une quelconque des revendications précédentes, dans lequel le procédé comprend la détermination quantitative du nitrite (NO₂⁻), du nitrate (NO₃⁻), de la mélamine (C₃H₆N₆), de l'urée ((NH₂)₂CO), de NH₃, NH₄⁺, ou de toutes associations de ceux-ci.

5. Procédé selon l'une quelconque des revendications précédentes, dans lequel le procédé comprend la détermination des quantités relatives du nitrate (NO₃⁻) par rapport à l'ammoniac (NH₃ et NH₄⁺) et/ou le procédé comprend la détermination des quantités relatives de NH₃ par rapport au NH₄⁺.

6. Procédé selon l'une quelconque des revendications précédentes, dans lequel le liquide aqueux est ou comprend un liquide corporel mammalien choisi parmi l'urine et/ou est le lisier.

7. Procédé selon l'une quelconque des revendications précédentes, dans lequel la lecture de RMN est effectuée dans un champ magnétique de jusqu'à environ 2,5 teslas.

8. Procédé selon l'une quelconque des revendications précédentes, dans lequel le liquide aqueux est sous la forme de l'extrait aqueux de sol d'un échantillon de sol, l'extrait aqueux de sol est obtenu par lavage de l'échantillon de sol avec une quantité préétablie d'eau, telle que de l'eau contenant in composant inorganique à ¹⁴N et/ou ³¹P connu, telle que de l'eau sensiblement pure, par exemple de l'eau obtenue par une filtration par osmose inverse.

9. Procédé selon la revendication 8, dans lequel le lavage de l'échantillon de sol avec de l'eau comprend le fait de mélanger l'échantillon de sol avec l'eau et de soumettre le mélange à une filtration pour éliminer par filtration une importante quantité en poids du sol, la filtration étant de préférence une microfiltration par exemple avec une limite de coupure d'environ 200 Da ou une filtration par osmose inverse.

10. Procédé selon l'une quelconque des revendications précédentes, le procédé comprenant le fait d'augmenter le rapport signal/bruit du spectre de données en soumettant le liquide aqueux à un traitement de polarisation au cours de la lecture de RMN, le traitement de polarisation comprend de préférence une DEPT (augmentation sans distorsion par transfert de polarisation) ou un NOE (effet Overhauser nucléaire).

11. Procédé selon l'une quelconque des revendications précédentes, dans lequel le procédé comprend en outre la détermination quantitative d'au moins un composant phosphoré et/ou la détermination quantitative d'au moins un ion contenant du phosphore inorganique.

12. Procédé selon l'une quelconque des revendications précédentes, dans lequel le procédé comprend en outre la détermination quantitative d'au moins un composant carboné, de préférence par soumission d'au moins une partie du liquide aqueux à une et/ou détermination quantitative de composants hydrocarbonés, de préférence par soumission d'au moins une partie du liquide aqueux à une lecture de RMN en combinaison avec une polarisation croisée de préférence sous la forme de DEPT comprenant la génération de données de ¹³C et corrélation des données de RMN-¹³C avec des données d'étalonnage, de préférence le procédé comprend en outre le fait d'effectuer une estimation quantitative des composés azotés organiques ayant une masse moléculaire d'au moins environ 200 Da dans le liquide aqueux en supposant une relation quantitative fixée de ¹³C ou de composants hydrocarbonés à ces composés azotés organiques.

13. Procédé selon l'une quelconque des revendications précédentes, dans lequel le procédé comprend en outre le fait de déterminer la quantité de H₂O dans le liquide aqueux et de déterminer ainsi la quantité de matière sèche, la quantité de H₂O est déterminée de préférence en effectuant au moins une lecture de RMN des noyaux ¹⁷O dans le liquide aqueux, la lecture comprend l'obtention de données de RMN-¹⁷O et la détermination de la quantité de H₂O dans un échantillon par corrélation des données de RMN-¹⁷O avec des données d'étalonnage, de préférence la teneur en azote en liaison organique est estimée par rapport à la teneur en matière sèche.

14. Procédé selon l'une quelconque des revendications précédentes, dans lequel la température mesurée réellement d'au moins une partie du liquide aqueux et les teneurs en NH₃ mesurées relativement par rapport au NH4⁺ sont utilisées pour calculer et afficher le pH dans l'intervalle de 5,5 à 12.
